Europäisches Patentamt

European Patent Office

Office européen des brevets

(11) Veröffentlichungsnummer: **0 174 379**
**B1**

(12) **EUROPÄISCHE PATENTSCHRIFT**

(45) Veröffentlichungstag der Patentschrift:
08.06.88

(21) Anmeldenummer: **84110197.5**

(22) Anmeldetag: **28.08.84**

(51) Int. Cl.⁴: **C 07 C  51/573,** C 07 C  57/145

(54) Verfahren zur Herstellung von Maleinsäureanhydrid.

(43) Veröffentlichungstag der Anmeldung:
**19.03.86 Patentblatt 86/12**

(45) Bekanntmachung des Hinweises auf die Patenterteilung:
**08.06.88 Patentblatt 88/23**

(84) Benannte Vertragsstaaten:
**AT BE CH DE FR GB IT LI NL**

(56) Entgegenhaltungen:
**DE-A-3 321 704**

(73) Patentinhaber: **HÜLS AKTIENGESELLSCHAFT, - RSP Patente / PB 15 - Postfach 13 20, D-4370 Marl 1 (DE)**

(72) Erfinder: **Gude, Fritz, Dr., Wilhelmstrasse 4, D-4690 Herne 2 (DE)**
Erfinder: **von Praun, Ferdinand, Dr., Am Schmöningsberg 8, D-4350 Haltern 6 (DE)**

LIBER, STOCKHOLM 1988

**Beschreibung**

Bei der Luftoxidation von Kolhenwasserstoffen, wie z. B. Benzol, Butan, Buten, Butadien usw., zu Maleinsäureanhydrid und Wasser bei 400°C fallen als Nebenprodukte in der Hauptsache noch niedrige Carbonsäuren, Kohlenoxid und Kohlendioxid an. Um möglichst viel Maleinsäureanhydrid unmittelbar aus dem Oxidationsstrom in fester Form gewinnen zu können, kühlt man i.a. den Luftstrom mit den erzeugten Substanzen zunächst auf Temperaturen oberhalb des Taupunktes des begleitenden Wasser zur Verhinderung der Hydrolyse ab. Dadurch ist es möglich, unmittelbar nach der Benzoloxidation bis zu etwa 85 Gew.-% und gleich nach Butan/Buten-Oxidation etwa 30-50 Gew.-% des insgesamt erzeugten Maleinsäureanhydrids abzuscheiden. Um die restliche Menge zu gewinnen, wäscht man den Maleinsäureanhydrid enthalteden Gasstrom mit soviel Wasser, daß das Anhydrid gerade vollständig niedergeschlagen wird. Dabei entsteht unter Hydrolyse eine etwa 40-%-ige Maleinsäurelösung. Um daraus wieder Maleinsäureanhydrid gewinnen zu können, wird in der Technik i.a. durch einen aufwendigen, energieverbrauchenden Prozeß unter Zugabe von Schleppmitteln, wie z. B. Xylol, das gesamte Wasser über den Kopf einer geeignten Kolonne abdestilliert, wobei sich das Maleinsäureanhydrid aus der Maleinsäure zurückbildet. Daneben entsteht durch cis-trans-Isomerisierung auch noch eine geringe Menge Fumarsäure. Selbstverständlich ist es auch möglich, auf die Abscheidung des Oxidationsstromes ganz zu verzichten und statt dessen das gesamt Oxidationsgas unmittelbar nach Reaktoraustritt mit Wasser auszuwaschen. Das kann vor allem nach der Oxidation von $C_4$ Gasen günstig sein, weil sich wegen des höheren Wassertaupunktes nur eine relativ geringe Menge Maleinsäureanhydrid durch Abkühlen des Oxidationsstromes direkt gewinnen läßt. Allerdings ist diese Arbeitsweise besonders energieaufwendig.

Neben anderen Nachteilen weisen die empfohlenen Lösungsmittel vor allem geringe Extraktionseffekte besonders unter den Verfahrensbedingungen auf, die dadurch bewirkt werden, daß der große Oxidationsgasstrom das Maleinsäureanhydrid aus dem organischen Medium wieder weitgehend entfernt.

Offensichtlich führt also Extraktion des Maleinsäureanhydrids mit indifferenten organischen Medien bei dem Luftoxidationsverfahren nur zu einem bescheidenen Erfolg. Dagegen sollten sich die durch Hydrolyse durch Hydrolyse oder Alkoholyse des Maleinsäureanhydrids entstehenden Carboxylgruppen an geeignete basische Extraktionsmittel intensiver binden. Um überschüssiges Wasser abtrennen zu könnnen, darf das Extraktionsmittel nicht darin löslich sein. Ferner soll durch einen einfachen Prozeß - möglichst nur durch Erhitzen des Extraktes - sich der Verbund mit der Maleinsäure lösen und nach Bildung des Maleinsäureanhydrids eine saubere Trennung auch vom entstandenen Wasser erfolgen. Das Extraktionsmittel muß erneut verwendbar sein. Ein von M.I. Jakuschkin (SU-PS-168 674) beschriebenes Verfahren behandelt die Isolation von niedrigen Monocarbonsäuren wie Ameisen-, Essig-, Propion- und Buttersäure, durch Extraktion aus wässrigen Lösung mit dem wasserunlöslichen Trioctylamin. Aus dem gebildeten Salz können nach Abtrennung des Wasser durch thermische Behandlung die Säuren in Freiheit gesetzt werden.

Es stellte sich nun heraus, daß sich auch Maleinsäure, also eine Dicarbonsäure, mit Tri-n-octylamin aus wässrigen Lösungen extrahieren läßt. Als prohibitiv für zwei der drei weiter unten beschriebenen Verfahrenswege erwies sich aber die mangelnde Stabilität des Tri-n-octylamins gegenüber dem Luftsauerstoff des Oxidationsgases. Auf der Suche nach unter den Betriebsbedingungen gegenüber einer großen Menge Luft resistenten, wasserunlöslichen tertiären Aminen wurde gefunden, daß überraschenderweise bestimmte verzweigte Kohlenstofketten die Stabilität der Base stark erhöhen. So überstand z. B. Tri-(2-ethylhexyl)-amin wochenlanges Durchperlen von Luft, auch bei Abwesenheit von Oxidationsinhibitoren, praktisch ohne Abbau.

Eine der Verfahrensweisen stellt sich nun folgendermaßen dar: Der Luftstrom aus dem Reaktor mit den erzeugten Substanzen wird, gegebenenfalls nach auf eine Temperatur kurz oberhalb des Wassertaupunktes zwecks Abscheidung des Maleinsäureanhydrids, nun unterhalb des Taupunktes des Wassers, gegebenenfalls nach Zugabe von weiterem Wasser, in ein flüssiges, wasserunlösliches, gegen Luft stabiles, tertiäres Amin - wie z. B. Tri-(2-ethylhexyl)-amin - geleitet. Die tertiäre Base ist mit indifferenten Kohlenwasserstoffen, vorzugsweise aromatischen Kohlenwasserstoffen, verdünnt. Durch das niedergeschlagene Wasser hydrolysiert Maleinsäureanhydrid zu Maleinsäure, die durch das tertiäre Amin gebunden wird. Das gebildete ölige Salz ist ebenfalls in Wasser unlöslicht. Nach Abtrennen des Wassers von der organischen Schicht überführt man nun die Amin-Maleinsäureadditionsverbindung in eine Destillationsapparatur. Beim Erhitzen auf etwa 120-180°C unter Vakuum tritt eine Spaltung in Amin, Maleinsäureanhydrid und auch Wasser ein, das durch Zugabe eines Schleppmittels abgetrennt wird. Das Maleinsäureanhydrid wird durch Abdestillation rein gewonnen. Zweckmäßig verwendet man für diesen Prozeß eine Apparatur, wie sie z. B. in "Ullmanns Encyklopädie der technischen Chemie", 4. Auflage, Bd. 16, Seite 407-411, beschrieben ist. Wie sich herausstellte, erfolgt nach der destillativen Entfernung des Schleppmittels eine Phasentrennung zwischen Maleinsäureanhydrid und Amin oberhalb 50°C. Zur schonenderen Aufarbeitung kann nun nach Separierung das Maleinsäureanhydrid durch Destillation gereinigt werden, während das Amin unmittelbar in den Extraktionsprozess zurückgeführt werden kann.

Alternativ zu dieser Arbeitsweise kann nach der Oxidation - gegebenenfalls nach Abtrennung eines Teiles des Maleinsäureanhydrids durch Kühlung auf eine Temperatur oberhalb des Taupunktes des begleitenden Wassers - das Luft-Wasser-Maleinsäureanhydrid-Gemisch ebenfalls oberhalb des Wassertaupunktes durch ein Gemisch eines geeigneten hochsiedenden Alkohols mit dem tertiären Amin geleitet werden. Dabei bildet sich unter Ringöffnung des Maleinsäureanhydrids ein Halbester, während die Carboxylgruppe unter Salzbildung mit dem Amin reagiert. Auf diese Weise wird praktisch die gesamte Maleinsäureanhydridmenge aus dem Luftstrom gewaschen. Durch Erhitzen dieser trockenen Waschflüssigkeit auf etwa 160°C und einem schwachen Vakuum tritt Spaltung der Komponenten unter Destillation des Maleinsäureanhydrids ein. Dabei können sich Diester des verwendeten Alkohols als Nebenprodukt bilden. Als Alkohol sind besonders oberhalb 220°C siedende Alkohole oder deren Gemische geeignet, wie z. B. Dekanol, Isodekanol, Laurylalkohol, Myristylalkohol, Palmitylalkohol, Butyldiglykol usw. Die Oxidation wird durch das Amin gegenüber der Luft inhibiert. Endlich kann man im Sinne dieser Erfindung auch zunächst bei dem üblichen Verfahren bis vor dem Abdestillieren des Wassers nach der Wasserwäsche des das Maleinsäureanhydrid enthaltenden Gasstroms bleiben. Durch die Wasserwäsche hydrolysiert das Maleinsäureanhydrid zu Maleinsäure. Um sich nun den hohen Energieaufwand beim Abdestillieren des Wassers zu ersparen, extrahiert man die Maleinsäure mit wasserunlöslichen tertiären Aminen. Bei dieser Verfahrensweise können neben den bereits erwähnten verzweigten tertiären Aminen nun auch sauerstoffempfindliche tertiäre Amine wie z. B. Tri-n-hexylamin, Tri-n-octylamin, Tri-n-dodecylamin, Tri-(3.5.5.-trimethylhexyl)-amin, Tri-(3.5.5.-trimethyloctyl)-amin, Tri-(3.5.5-trimethyldecyl)-amin, N,N-Dioctyl-(2-ethylhexyl)-amin, N-Octyl-N-(4-heptyl)-(2-ethylhexyl)-amin, N-Octyl-N-(4-heptyl)-cyclohexylamin, N-Octyl-N-(2-ethylhexyl)-cyclohexylamin, N-Dodecyl-3.3.5-trimethyl-azacyclo-heptan, N,N-Dioctylanilin usw. eingesetzt werden. Die Aufarbeitung des gebildeten Salzes erfolgt wie vorher durch Abdestillation des Maleinsäureanhydrids, nachdem das durch Zugabe eines Schleppmittels entstandene Wasser azeotrop übergegangen ist.

Als Extraktionsmittel eignen sich alle wasserunlöslichen, unter den Reaktionsbedingungen flüssigen, tertiären Amine mit einem $pK_A$-Wert unter 9, deren Salze Maleinsäure ebenfalls wässerunlöslich sein müssen. Wenn direkt aus den großen Menge Luft enthaltenden Reaktionsgasen extrahiert werden soll, wird der Einsatz von unter den Reaktionsbedingungen gegen Luft stabilen verzweigten tertiären Amin mit in 2-Stellung verzweigten primären aliphatischen Seitenketten wie z. B. Tri-(2-ethylhexyl)-amin, Tri-(2-ethylbutyl)-amin oder Tri-(2-ethyldecyl)-amin usw. empfohlen. Die als Blasenrückstand nach dem Abdestillieren des Maleinsäureanhydrids verbleibenden tertiären Amine konnten ohne Reinigung für nachfolgenden Chargen mehrfach verwendet werden.

Die Salze aus Maleinsäure und tertiären Aminen mit in 2-Stellung verzweigten primären aliphatischen Seitenketten spalten sich bei thermischer Belastung etwa 30°C früher in ihre Komponenten als die der Maleinsäure mit unverzweigten tertiären Aminen. Die bei niedrigen Temperatur eintretende Freisetzung der Maleinsäure schont die Substanzen und erhöht insbesonderen die Ausbeuten an gewinnbarem Maleinsäureanhydrid auf annähernd quantitative Wert. Aus diesen Grunde wird der Einsatz von tertiären Aminen mit in 2-Stellung verzweigten primären aliphatischen Seitenketten grundsätzlich bevorzugt.

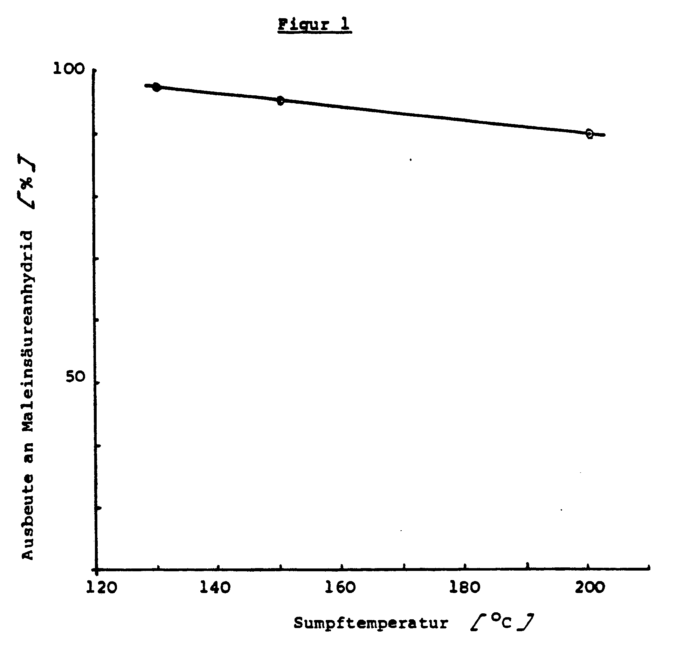

Die z. B. in der GB-PS-933 102 beschriebene spontane Umwandlung des Maleinsäureanhydrids sowie seiner Halbester und Polymerisation der entstandenen Reaktionsprodukte unter der katalytischen Wirkung von tertiären Aminen konnte in dem vorliegenden Verfahren bei den erfindungsgemäßen Bedingungen mit den genannten tertiären Aminen nicht bestätigt werden, wie die weiter unten aufgeführten Beispiele belegen. Selbstverständlich ist es zur Schonung der Substanzen und damit zur Erhöhung der Maleinsäureanhydridausbeute sowie der Sauberhaltung des tertiären Amins nützlich, die Sumpftemperatur bei der Destillation möglichst niedrig zu halten. So konnte beim Einsatz von Tri-(2-ethylhexyl)-amin als Extraktionsmittel 98 % der Theorie des extrahierten Maleinsäureanhydrids durch Destillation bei 15 mbar (Sumpftemperatur etwa 130°C) gewonnen werden. Die Abhängigkeit der Ausbeute an Maleinsäureanhydrid in Tri-(2-ethylhexyl)-amin von der Blasentemperatur bei kontinuierlicher Destillation zeigt die grafische Auftragung in Figur 1.

Die Kombination der zum Teil bekannten Verfahrensstufen zur energiesparenden Herstellung von Maleinsäureanhydrid ist neu.

Gegenstand dieses Schutzrechtes ist daher ein Verfahren zur Hertstellung von Maleinsäureanhydrid durch katalytische Luftoxidation von geeigneten Kohlenwasserstoffen, wie z. B. Buten, Butan, Butadien oder Benzol und/oder deren Gemischen bei Temperaturen von 300-450°C, das gekennzeichnet ist durch die folgenden, sich an die Luftoxidation anschließenden Schritte:

1. gegebenenfalls teiweises Abscheiden des Maleinsäureanhydrids durch Abkühlung auf Temperaturen oberhalb des Taupunktes des begleitenden Wassers

2. Einleiten des Maleinsäureanhydrid und Wasserdampf enthaltenden Luftstromes

a) unterhalb des Taupunktes des begleitenden Wasser, wobei gegebenenfalls weiteres Wasser zugesetzt wird, in ein in Wasser unlösliches, gegen Luft stabiles tertiäres Amin, das noch einen der üblichen Kohlenwasserstoffe als Schleppmittel für das Wasser enthält und Separieren der organischen Schicht oder

b) oberhalb des Taupunktes des begleitenden Wassers in ein Gemisch von in Wasser unlöslichem, gegen Luft stabilem, tertiärem Amin und einem Alkohol geringen Dampfdrucks und Abtrennen der organischen Schicht oder

c) in Wasser und anschließende Extraktion der Maleinsäure mit einem in Wasser unlöslichen tertiärem Amin, das einen der üblichen Kohlenwasserstoffe als Schleppmittel für das Reaktionswasser enthält und Separieren der organischen Schicht, wobei die als Extraktionsmittel eingesetzen, in Wasser unlöslichen tertiären Amin einen $pK_A$-Wert < 9 besitzen und im Falle der Verfahrensschritte 2a und 2b in 2-Stellung verzweigte, primäre aliphatische Seitenketten tragen.

3. Erhitzen der nach 2.) erhalten Extrakte auf Temperaturen von 120-180°C unter Vakuum zur Gewinnung des Maleinsäureanhydrids.

### Beispiele

1) 1000 g einer 29,6-%-igen wäßrigen Maleinsäurelösung wurden in einem einmaligen Extraktionsschritt mit einer Mischung aus 1500 g Tri-(2-ethylhexyl)-amin und 1500 g Xylol intensiv ausgeschüttelt. Der organische Extrakt enthielt danach 94 % der eingesetzen Maleinsäure. Er wurde an Wasserabscheider zum Sieden erhitzt und schied bei einer Sumpftemperatur von 130°C und einem Druck von 700 mbar 105 ml Wasser ab. Der Rückstand wurde anschließend einer Vakuumdestillation unterworfen. Nach destillativer Entfernung des Schleppmittels gingen bei 100-105°C/15 mbar 230,4 g Maleinsäureanhydrid über, entsprechend einer Ausbeute von 98 % d.Th. Im Sumpfaustrag, der in wesentlichen das hochsiedende Amin enthielt, konnten noch geringe Menge Fumarsäure nachgewiesen werden. Der Sumpf kann ohne jede Reinigung für weitere Maleinsäureextraktionen erneut zum Einsatz kommen.

2-5) 1000 g einer 34,7-%-igen wäßrigen Maleinsäurelösung wurden dreimal mit einer Mischung von 1000 g Amin und 1000 g Diethylbenzol 15 Minuten lang durch Ausrühren extrahiert. Aus dem maleinsäurehaltigen Extrakt wurden am Wasserabscheider bei einer Sumpftemperatur von 160-165°C und einem Druch von 300 mbar zwishen 140 und 170 ml Wasser abgetrennt. Der Rückstand wurde anschließend destillativ aufgearbeitet und lieferte bei 100-105°C/16 mbar siedendes Maleinsäureanhydrid.

| Amin | Extraktionsrate (%) | Spaltrate (%)[+] |
|---|---|---|
| Tri-n-dodecylamin | 99,7 | 92,8 |
| Tri-n-octylamin | 99,8 | 70,0 |
| N-Dodecyl-3.3.5-tri-methylazacycloheptan | 98,8 | 54,8 |
| N,N-Dioctylanilin | 88,0 | 59,1 |

[+] Spaltrate = Ausbeute an Maleinsäureanhydrid

6) Bei der Herstellung von Maleinsäureanhydrid durch katalytische Luftoxidation von Kohlenwasserstoffen, wie z. B. Benzol, wird das Reaktionsgas zunächst durch einen Partial-Kondenser geleitet, in dem ein Teil des Anhydrids abgetrennt wird. Das den Abscheider verlassende Abgas wurde nun zur Gewinnung der Restmenge an Maleinsäureanhydrid in einer Extraktionskolonne mit einer Mischung aus ca. 50 Gew.-% Tri-(2-ethylhexyl)-amin und 50 Gew.-% Xylol im Gegenstrom behandelt, und zwar unterhalb des Taupunktes des begleitenden Wassers (ca. 30°C).

3035 g eines hierbei gewonnenen Extraktes im wesentlichen bestehend aus 1500 g Amin, 1395 g Xylol und 90 g Maleinsäure wurden pro Stunde in die obere Hälfte einer Destillationskolonne eingefahren, die mit einer Sumpftemperatur von 130°C bei einem Druck von 700 mbar betrieben wurde. Am Kopf der Kolonne fielen stündlich in azeotroper Mischung mit Xylol 54 g Wasser an, während das Sumpfprodukt aus Amin, Xylol und Maleinsäureanhydrid unten abgezogen und einer zweiten Kolonne zur Reindestillation zugeführt wurde. Hier wurde das restliche Schleppmittel am Kolonnenkopf abdestilliert und 75 g/h reines Maleinsäureanhydrid im Seitenstrom der Kolonne bei einer Temperatur von 160°C und einem Druck von 200 mbar isoliert. Die Ausbeute an Maleinsäureanhydrid bezogen auf die Thermolyse beträgt 98,6 % d.Th. Das im Sumpf befindliche Amin wurde nach Reiningung zusammen mit dem abgetrennten Xylol in die Extraktion zurückgefahren.

7) Das Abgas aus der Maleinsäureanhydridherstellung (vgl. Beispiel 6) wurde in einer Extraktionskolonne mit einer Mischung aus etwa 30 % Tri-(2-ethylhexyl)-amin, 18 % Laurylalkohol und 52 % Dodecan im Gegenstrom bei einer Temperatur oberhalb des Taupunktes des begleitenden Wassers behandelt.

2950 g des hierbei anfallenden Extraktes vorwiegend aus 104 g Amin, 1272 g Aminsalz des Maleinsäurelaurylhalbesters, 120 g Laurylalkohol und 1400 g Dodecan wurden pro Stunde auf die Kolonne einer kontinuierlich arbeitenden Destillationsapparatur gefahren, die mit einer Sumpftemperatur von 160-170°C bei einem Druck von 200 mbar betrieben wurde. Am Kopf der Kollone fiel stündlich ein Gemisch aus 176 g Maleinsäureanhydrid und 840 g Dodecan an, das sich bei 55-60°C in zwei flüssige Phasen trennte. Die obere Dodecanphase wurde rezirkuliert und die untere Anhydridphase noch destillativ gereinigt. Das Sumpfprodukt aus der thermischen Spaltung, bestehend aus Amin, Dodecan und Laurylalkohol enthielt noch Maleinsäuredilaurylester, der vor Rückführung der Mischung in die Extraktionsstufe abfiltriert wurde. Ausbeute an Maleinsäureanhydrid bei Halbesterthermolyse: 90 % d.Th.

### Patentansprüche

1. Verfahren zur Herstellung von Maleinsäureanhydrid durch katalytische Luftoxidation von geeigneten Kohlenwasserstoffen, wie z. B. Buten, Butan, Butadien oder Benzol und/oder deren Gemischen bei Temperaturen von 300-450°C gekennzeichnet durch die folgenden, sich an die Luftoxidation anschließenden Schritte

I. gegebenenfalls teilweises Abscheiden des Maleinsäureanhydrids durch Abkühlung auf Temperaturen oberhalb des Taupunktes des begleitenden Wassers,

II. Einhelten des Maleinsäureanhydrid und Wasserdampf enthaltenden Luftstromes

a) unterhalb des Taupunktes des begleitenden Wassers, wobei gegebenenfalls weiteres Wasser zugesetzt wird, in ein in Wasser unlösliches, gegen Luft stabiles tertiäres Amin, das noch einer der üblichen Kohlenwasserstoffe als Schleppmittel für das Wasser enthält, und Separieren der organischen Schicht
oder

b) oberhalb des Taupunktes des begleitenden Wasser in ein Germisch von in Wasser unlöslichem, gegen Luft stabilem tertiärem Amin und einem Alkohol geringen Dampfdrucks und abtrennen der organischen Schicht
oder

c) in Wasser und anschließende Extraktion der Maleinsäure mit einem in Wasser unlöslichen tertiärem Amin, das einen der üblichen Kohlenwasserstoffe als Schleppmittel für das Reaktionswasser enthält, und Separieren der organischen Schicht

III. Erhitzen der nach II erhaltenen Extrakte auf Temperaturen von 120-180°C unter Vakuum zur Gewinnung des Maleinsäureanhydrids, wobei die als Extraktionsmittel eingesetzten, in Wasser unlöslichen tertiären Amine einen $pK_A$-Wert < 9 besitzen und im Falle der Verfahrensschritte IIa und IIb in 2-Stellung verzweigte, primäre aliphatische Seitenketten tragen.

2. Verfahren zur Herstellung von Maleinsäureanhydrid nach Anspruch 1,
dadurch gekennzeichnet,
daß für Verfahrensschritt IIc tertiäre Amin, die in 2-Stellung verzweigte, primäre aliphatische Seitenketten tragen, eingesetzt werden.

3. Verfahren nach den Ansprüchen 1 und 2,
dadurch gekennzeichnet,
daß als Amin Tri-(2-ethylhexyl)-amin eingesetzt wird.

## Claims

1. Process for preparing maleic anhydride by catalytic air oxidation of suitable hydrocarbons, such as, for example, butene, butane, butadiene or benzene and/or their mixtures at temperatures of 300-450°C, characterized by the following stages which follow the air oxidation

I. if necessary, partial precipitation of maleic anhydride by cooling to temperatures above the dew point of accompanying water,

II. leading the air stream containing the maleic anhydride and steam

(a) at a temperature below the dew point of accompanying water, if necessary adding more water, into a water-insoluble tertiary amine, which is stable in air, which also contains one of the usual hydrocarbons as entrainer for water, and separating the organic layer
or

(b) at a temperature above the dew point of accompanying water, into a mixture of a water-insoluble tertiary amine, which is stable in air, and an alcohol which has a low vapour pressure, and separating the organic layer
or

(c) into water, then extracting the maleic acid with a water-insoluble tertiary amine, which contains one of the usual hydrocarbons as entrainer for the reaction water, and separating the organic layer

III. heating the extracts obtained in II to 120-180°C under vacuum to recover the maleic anhydride, whereby the water-insoluble tertiary amines used as extractant have a $pK_A$ value < 9, and in the case of stages IIa and IIb have a primary aliphatic side chain branched at position 2.

2. Process for preparing maleic anhydride according to Claim 1, characterized in that for process stage IIc, tertiary amines with primary aliphatic side chains branched at position 2 are used.

3. Process according to Claims 1 and 2 characterized in that tri-(2-ethylhexyl)-amine is used as amine.

## Revendications

1. Procédé pour l'obtention d'anhydride maléïque par oxydation à l'air catalytique d'hydrocarbures appropriés comme par exemple Butène, Butane, Butadiène ou Benzène et/ou leurs mélanges au moyen des étapes suivantes associées à l'oxydation à l'air:

I. éventuellement séparation partielle de l'anhydride maléïque par refroidissement à des températures au-dessus du point de rosée de l'eau accompagnante,

II. introduction du courant d'air contenant l'anhydride maléïque et la vapeur d'eau,

a) en-dessous du point de rosée de l'eau accompagnante, pour laquelle éventuellement davantage d'eau est ajoutée, dans une amine tertiaire stable vis-à-vis de l'air, insoluble, dans l'eau qui contient en plus un des hydrocarbures usuels comme agent d'entraînement pour l'eau et séparation de la couche organique ou

b) au-dessus du point de rosée de l'eau accompagnante dans un mélange d'une amine tertiaire stable vis-à-vis de l'air insoluble dans l'eau et d'un alcool de faible tension de vapeur, et séparation de la couche organique ou

c) dans l'eau et ensuite extraction de l'acide maléïque avec une amine tertiaire insoluble dans l'eau qui contient un des hydrocarbures usuels en tant qu'agent d'entraînement pour l'eau réactionnelle et séparation de la couche organique.

III. Chauffage de l'extrait obtenu selon II à des températures de 120 à 180°C, sous vide, pour obtenir l'anhydride maléïque par lequel les amines tertiaires insolubles dans l'eau mises en oeuvre en tant que moyen d'extraction possèdent une vapeur de pKa < 9 et dans le cas des étapes du procédé IIa et IIb portent des chaînes latérales aliphatiques, primaires, ramifiées en position 2.

2. Procédé pour l'obtention de l'anhydride maléïque selon la revendication 1, caractérisé en ce que des amines tertiaires pour l'étape du procédé IIc qui portent des chaînes latérales aliphatiques primaires ramifiées en position 2, sont mises en oeuvre.

3. Procédé selon les revendications 1 et 2, caractérisé en ce que comme amine la tri-(2-éthylhexyl)amine est mise en oeuvre.

## Figur 1